(19) 

**Europäisches Patentamt**
**European Patent Office**
**Office européen des brevets**

(11) **EP 1 852 064 A1**

(12) **EUROPEAN PATENT APPLICATION**

(43) Date of publication:
**07.11.2007 Bulletin 2007/45**

(51) Int Cl.:
***A61B 5/028*** (2006.01)

(21) Application number: **07008877.8**

(22) Date of filing: **02.05.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK YU**

(30) Priority: **05.05.2006 DE 102006021034**

(71) Applicant: **UP Management GmbH & Co Med-Systems KG**
**81673 München (DE)**

(72) Inventors:
• **Michard, Frederic, Dr.**
  **91570 Biévres (FR)**
• **Pfeiffer, Ulrich, Dr.**
  **81667 Munich (DE)**
• **Knoll, Reinhold**
  **81543 Munich (DE)**

(74) Representative: **DTS München**
**St.-Anna-Strasse 15**
**80538 München (DE)**

(54) **Apparatus and computer program for assessing a patient's extravascular lung water volume**

(57)    An apparatus for assessing extravascular lung water volume (EVLW) of a patient after lung resection is adapted to provide a transpulmonary thermodilution curve, is capable to derive the patient's intrathoracic thermal volume (ITTV) and approximated intrathoracic blood volume (ITBV$_{approx}$) from the transpulmonary thermodilution curve, and is capable to determine the extravascular lung water volume (EVLW) by correcting by the degree of lung resection (c), wherein making use of the equation EVLW = f(ITTV, ITBV$_{approx}$, c), or EVLW = f(ITTV, GEDV, c). Further, a computer program for assessing extravascular lung water volume (EVLW) of a patient after lung resection, has instructions adapted to carry out the steps of generating a transpulmonary thermodilution curve on basis of provided measurement data collected at the patient, deriving the patient's intrathoracic thermal volume (ITTV) and approximated intrathoracic blood volume (ITBV$_{approx}$) from the transpulmonary thermodilution curve, and determining the extravascular lung water volume (EVLW) by correcting by the degree of lung resection (c), wherein making use of the equation EVLW = f(ITTV, ITBV$_{approx}$, c) or EVLW = f(ITTV, GEDV, c), when run on a computer.

FIG. 1

**Description**

Field of the invention

The invention relates to an apparatus and a computer program for observing a patient's life-threatening condition caused by pulmonary edema by assessing the patient's extravascular lung water volume.

Background of the Invention

It is generally known that a patient suffering from pulmonary edema is in a life threatening condition. Such a pulmonary edema may result from an increase in hydrostatic pulmonary capillary pressure (cardiogenic pulmonary edema) and/or from an increase in pulmonary capillary permeability (acute lung injury, Acute Respiratory Distress Syndrome).

In the critical-care diagnostics and treatment of such a patient it is useful to use the patient's extravascular lung water volume EVLW as an important characteristic for monitoring the patient's state of health to guide therapy and to improve the outcome thereof.

In general, a normal value of extravascular lung water volume EVLW of a healthy person ranges between 3 and 7 ml/kg. However, a patient suffering from an increase in hydrostatic pulmonary capillary pressure (cardiogenic pulmonary edema) and/or from an increase in pulmonary capillary permeability (acute lung injury, Acute Respiratory Distress Syndrome) leading to a pulmonary edema may has a value of extravascular lung water volume EVLW reaching 25 to 30 ml/kg.

For accurate determination of extravascular lung water volume EVLW of an animal the usage of an ex-vivo post-mortem gravimetric method is known. By nature this method can only be employed once and, of course, is not applicable in humans for repeated monitoring of EVLW.

According to findings in literature, for purposes of monitoring the extravascular lung water volume EVLW can repeatedly be derived from the difference between the intrathoracic thermal volume ITTV and the intrathoracic blood volume ITBV of the patient, i.e.

$$EVLW = ITTV - ITBV.$$

The intrathoracic thermal volume ITTV and the intrathoracic blood volume ITBV can be determined using a transpulmonary thermodilution analysis method, as it is proposed in US 5,526,817. A bolus of an indicator defined by a predetermined quantity of the indicator is injected into the patent's vena cava superior, and the indicator concentration response is measured at a downstream location of the patient's systemic circulation, e.g. at the patient's femoral artery. Based on the indicator concentration response measurement versus time the transpulmonary thermodilution curve is generated. The intrathoracic thermal volume ITTV and the Global End Diastolic Volume GEDV are derived from the mathematical analysis of the transpulmonary thermodilution curve according to methods known from prior art. It has been established that the intrathoracic blood volume ITBV and the global end-diastolic volume GEDV are closely related and that the intrathoracic blood volume ITBV can be approximated from the global end-diastolic volume GEDV by using the equation

$$ITBV_{approx} = a\ GEDV + b.$$

The extravascular lung water volume EVLW can therefore be derived from the difference between the intrathoracic thermal volume ITTV and the approximated intrathoracic blood volume ITBV of the patient, i.e.

$$EVLW = ITTV - ITBV_{approx}.$$

It is desirable that above mentioned method for assessing the extravascular lung water volume EVLW of a patient is accurate and hence reliable for monitoring the patient's state of health to guide therapy and to improve the outcome thereof when applying such method to a patient suffering from pulmonary edema and having parts of his/her lung resected. However, the circumstance of lung resection affects the accuracy of results obtained by a transpulmonary thermodilution method. Therefore, when using a thermodilution method for determining a value of the patient's extravascular lung water volume EVLW for diagnostics and therapy, this value is inaccurate and hence only less significant in the clinical-care point of view.

It is an object of the invention to provide an apparatus and a computer program for observing a patient's life-threatening condition caused by pulmonary edema by assessing the patient's extravascular lung water volume in simple, but nevertheless exact and reliable way, although the patient has a resected lung.

Summary of the invention

According to the invention, this object is achieved by an apparatus for assessing extravascular lung water volume EVLW of a patient after lung resection, adapted to provide a transpulmonary thermodilution curve, capable to derive the patient's intrathoracic thermal volume ITTV and approximated intrathoracic blood volume $ITBV_{approx}$ from the transpulmonary thermodilution curve, and capable to determine the extravascular lung water volume EVLW by correcting said approximated intrathoracic blood volume $ITBV_{approx}$ by the degree of lung resection c, wherein making use of the equation

$$EVLW = f(ITTV, ITBV_{approx}, c),$$

or

$$EVLW = f(ITTV, GEDV, c).$$

Further, according to the invention, this object is achieved by a computer program for assessing extravascular lung water volume EVLW of a patient after lung resection. The computer program has instructions adapted to carry out the steps of generating a transpulmonary thermodilution curve on basis of provided measurement data collected at the patient, deriving the patient's intrathoracic thermal volume ITTV and approximated intrathoracic blood volume $ITBV_{approx}$ from the transpulmonary thermodilution curve, and determining the extravascular lung water volume EVLW by correcting said approximated intrathoracic blood volume $ITBV_{approx}$ by the degree of lung resection c, wherein making use of the equation

$$EVLW = f(ITTV, ITBV_{approx}, c),$$

or

$$EVLW = f(ITTV, GEDV, c),$$

when run on a computer.

Due to the fact that according to the invention the extravascular lung water volume EVLW is corrected on basis of the degree of lung resection c of the patient, the value of the extravascular lung water volume EVLW assessed by the inventive apparatus and computer program is accurate and reliable, although the value of the extravascular lung water volume EVLW is determined by making use of the easily feasible bedside method of thermodilution.

Preferably, the apparatus is adapted to estimate the degree of lung resection c by making use of an empirical average value of the reduction of pulmonary perfusion of the resected lung compared to the pre-operative lung of the patient. Further, according to a preferred embodiment of the invention, the computer program has instructions being adapted to carry out the step of this estimate.

As an alternative, it is preferred that the apparatus and the computer program make use of, in case of a resected right lung, estimating the degree of lung resection c as being 55%, or, in case of a resected left lung, estimating the degree of lung resection c as being 45%, and/or, in case of resected lobes, estimating the degree of lung resection c as being the sum of normal perfusion rates of the resected lobes.

Normal perfusion rate right lung:

    Right superior lobe 18%
    Right middle lobe 12%
    Right inferior lobe 25%

Normal perfusion rate left lung:

    Left superior lobe 25%
    Left inferior lobe 20%

As a further alternative, it is preferred that the apparatus is adapted to estimate the degree of lung resection c by comparing the pulmonary perfusion before and after the lung resection, wherein the pulmonary perfusion values are provided by making use of pre-operative perfusion lung scans and dividing the density of areas which will be resected by the density of the whole unresected lung. It is also a preferred alternative embodiment of the invention that the computer program has instructions adapted to carry out this estimate.

Preferably, the apparatus is adapted to make use of the equation

$$\mathtt{EVLW\ =\ ITTV\ -\ ITBV\Big|_{corrected'}}$$

wherein $\mathrm{ITBV}|_{corrected}$ is the value of the approximated intrathoracic blood volume $\mathrm{ITBV}_{approx}$ corrected by the degree of lung resection c. It is also preferred that the computer program has instructions adapted to carry out the step of making use of this equation.

It is preferred that the apparatus and the instructions of the computer program are adapted to make use of equating the degree of lung resection c with the degree of reduction of pulmonary blood volume PBV caused by the lung resection.

Further, the apparatus is adapted to derive the global end-diastolic volume GEDV of the patient from the transpulmonary thermodilution curve and is adapted to make use of the equation

$$\mathtt{ITBV\Big|_{corrected}\ =\ GEDV\ +\ PBV\Big|_{corrected'}}$$

wherein $\mathrm{PBV}|_{corrected}$ is the value of the pulmonary blood volume PBV corrected by the degree of lung resection c.

Preferably the computer program has instructions adapted to carry out the steps of deriving the global end-diastolic volume GEDV from the transpulmonary thermodilution curve and making use of the above equation.

Further, according to a preferred embodiment of the apparatus and the computer program, both are adapted to make use of the equation

$$\mathtt{PBV\Big|_{corrected}\ =\ ((a\ -\ 1)\ GEDV\ +\ b)\ (100\%\ -\ c[\%]),}$$

wherein a and b are parameters in particular set to be

$$\mathtt{a\ =\ 1.25\ and\ b\ =\ 0.0.}$$

Brief description of the Drawing

In the following the invention is explained on the basis of a preferred embodiment with reference to the drawing.

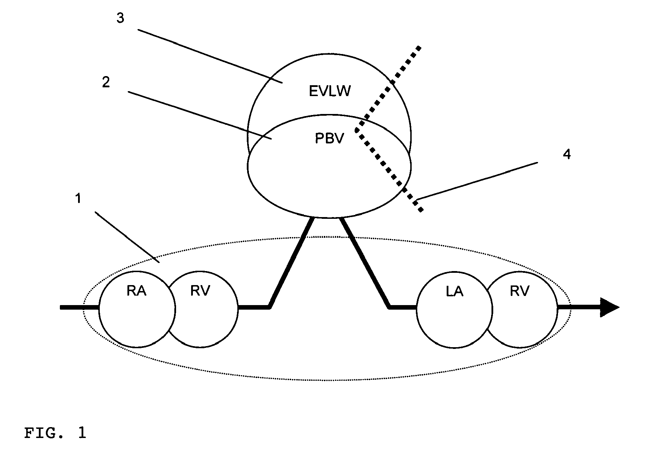

In the drawing shows Fig. 1 a model of a pulmonary circulation.

Detailed Description of a Preferred Embodiment of the Invention

**Fig. 1** shows a model of a pulmonary circulation comprising a heart and a lung. The right atrium RA of the heart, the right ventricle RV of the heart, the left atrium LA of the heart and the left ventricle LV of the heart define the sum of blood volumes outside the lung. This sum of blood volumes outside the lung defines the global end-diastolic volume GEDV designated with 1.

Further, the model of the pulmonary circulation comprises the pulmonary blood volume PBV designated with 2, and the extravascular lung water EVLW designated with 3.

To assess the extravascular lung water volume EVLW, a transpulmonary thermodilution method is used. This method is based on the physiologic relationship between the global end-diastolic volume GEDV and the intrathoracic blood volume ITBV. The global end-diastolic volume GEDV is derived from the mathematical analysis of the transpulmonary thermodilution curve.

It has been established that the intrathoracic blood volume ITBV and the global end-diastolic volume GEDV are closely related and that the intrathoracic blood volume ITBV can be approximated from the global end-diastolic volume GEDV by using the equation

$$ITBV_{approx} = a\ GEDV + b,$$

wherein a and b are parameters, for example set to be a = 1.25 and b = 0.0.

The intrathoracic blood volume ITBV can be approximated from the global end-diastolic volume GEDV measurements. Therefore, the extravascular lung water volume EVLW can be estimated as the difference between the intrathoracic thermal volume ITTV and the approximated intrathoracic blood volume $ITBV_{approx}$.

However, when a patient undergoes lung resection, the accuracy of this approximation from transpulmonary thermodilution is affected by the lung resection.

In Figure 1 a lung resection cut which separates a part of the lung and of pulmonary blood volume PBV is shown and designated with 4.

The estimation of the extravascular lung water volume EVLW by the thermodilution method is based on the approximation of the intrathoracic blood volume ITBV from the global end-diastolic volume GEDV by using the equation

$$ITBV_{approx} = a\ GEDV + b.$$

Further, the approximated pulmonary blood volume $PBV_{approx}$ is defined by the difference between the approximated intrathoracic blood volume $ITBV_{approx}$ and the global end-diastolic volume GEDV, i.e.

$$PBV_{approx} = ITBV_{approx} - GEDV.$$

When taking above equations into account, the pulmonary blood volume PBV can be expressed by

$$PBV_{approx} = a\ GEDV + b - GEDV = b + (a - 1)\ GEDV.$$

In case of lung resection, the pulmonary blood volume PBV is actually much lower than predicted by this equation. Therefore, in particular this decrease in pulmonary blood volume PBV affects the estimation of the intrathoracic blood volume ITBV and hence the estimation of the extravascular lung water volume EVLW.

Taking above equations into account, the intrathoracic blood volume ITBV is calculated as

$$ITBV_{approx} = a \ GEDV + b = GEDV + PBV_{approx}$$
$$= GEDV + b + (a - 1) \ GEDV,$$

wherein [b + (a - 1) GEDV] represents the approximated pulmonary blood volume $PBV_{approx}$.

Most patients undergoing a lung resection are evaluated preoperatively by a perfusion lung scan allowing a quantification of the actual perfusion of each lung (or lung lobe). From this distribution of pulmonary perfusion the relative perfusion of the removed part of the lung can be estimated. For example, if 55% of pulmonary perfusion was going to the right lung and 45% of the pulmonary perfusion to the left lung, it can be assumed that after resection of the right lung the pulmonary blood volume will be decreased by 55%.

This assumption can be done in a similar way for each of the three right lung lobes and each of the two left lung lobes. Therefore, the decrease in pulmonary blood volume PBV after the lobe resection can be also predicted. For example, if it is known from the preoperative perfusion lung scan that the inferior right lobe collects 25% of the pulmonary perfusion, it could be assumed that inferior right lobe surgical resection will reduce the pulmonary blood volume by 25%. This expected reduction of pulmonary blood volume PBV in relation to the pre-operative pulmonary blood volume PBV could be used as degree of resection c of total pulmonary perfusion caused by the resection representing the lung or lung lobe removed during the surgical procedure.

In case a perfusion lung scan is not available (e.g. use of transpulmonary thermodilution of a patient who underwent lung resection surgery several years ago), post-operative pulmonary blood volume PBV and the degree of resection c can be estimated from the normal anatomical distribution of pulmonary blood volume in human beings.

In order to improve the calculation of the intrathoracic blood volume ITBV, the degree of resection c has to be taken into consideration.

In general, a corrected intrathoracic blood volume $ITBV|_{corrected}$ could be derived from the approximated intrathoracic blood volume $ITBV|_{approx}$ and the degree of resection c, i.e.

$$ITBV|_{corrected} = f(ITBV|_{approx}, \ c).$$

When using the transpulmonary thermodilution method known from US 5,526,817, the pulmonary blood volume PBV can be calculated as

$$PBV_{corrected} = (b + (a - 1) \ GEDV) \ x \ (100\% - c[\%])$$

and the intrathoracic blood volume ITBV can be calculated as

$$ITBV|_{corrected} = GEDV + PBV_{corrected} =$$
$$= GEDV + (b + (a - 1) \ GEDV) \ (100\% - c[\%]).$$

or as

$$ITBV|_{corrected} = ITBV|_{approx} - c[\%] \ (b + (a - 1) \ GEDV).$$

For example, if a = 1.25, b = 0.0, and c = 45% (e.g. surgical resection of a left lung representing 45% of the total pulmonary perfusion on a preoperative lung scan), the corrected intrathoracic blood volume $ITBV_{corrected}$ is calculated as

$$ITBV\big|_{corrected} = 1.1375 \; GEDV$$

Therefore, modifying the equation to derive $ITBV_{corrected}$ from GEDV according to the degree of resection c is easily settling the current limitation of the transpulmonary thermodilution method in estimating the extravascular lung water volume EVLW after lung resection.

Taking above mentioned and described modelling into account, a process for assessing extravascular lung water volume EVLW of a patient after lung resection comprises the steps of:

- Generating a transpulmonary thermodilution curve on basis of provided measurement data collected from the patient.

- Deriving the patient's intrathoracic thermal volume ITTV and approximated intrathoracic blood volume $ITBV_{approx}$ from the transpulmonary thermodilution curve based on relations derived with unresected lungs.

- Estimating the degree of lung resection c by making use of an empirical average value of the reduction of pulmonary perfusion of the resected lung compared to the pre-operative lung of the patient, or, in case of a resected right lung, estimating the degree of lung resection c as being 55%, or, in case of a resected left lung, estimating the degree of lung resection c as being 45%. Or estimating the degree of lung resection c by comparing the pulmonary perfusion before and after the lung resection, wherein the pulmonary perfusion values are provided by making use of pre-operative perfusion lung scans and dividing the density of areas which will be resected by the density of the whole unresected lung. Or in case of resected lobes, estimating the degree of lung resection c as being the sum of normal perfusion rates of the resected lobes.

Normal perfusion rate right lung:

Right superior lobe 18%
Right middle lobe 12%
Right inferior lobe 25%

Normal perfusion rate left lung:

Left superior lobe 25%
Left inferior lobe 20%

- Determining the corrected intrathoracic blood volume $ITBV_{corrected}$ by correcting for the degree of lung resection c, wherein making use of the equation

$$ITBV_{corrected} = f(ITBV_{approx}, \; c).$$

- Making use of the equation

$$EVLW = ITTV - ITBV\big|_{corrected},$$

wherein $ITBV\big|_{corrected}$ is the value of the intrathoracic blood volume ITBV corrected by the degree of lung resection c.

- Making use of equating the degree of lung resection c with the degree of reduction of pulmonary blood volume PBV caused by the lung resection.

- Deriving the global end-diastolic volume GEDV of the patient from the transpulmonary thermodilution curve.

- Making use of the equation

$$ITBV\big|_{corrected} = GEDV + PBV\big|_{corrected},$$

wherein $PBV\big|_{corrected}$ is the value of the pulmonary blood volume PBV corrected by the degree of lung resection c.

- Making use of the equation

$$PBV\big|_{corrected} = ((a - 1)\ GEDV + b)\ (100\% - c\%),$$

wherein a and b are parameters.

- Setting the parameters a and b to be
  a = 1.25 and b = 0.0.

**Claims**

1. Apparatus for assessing extravascular lung water volume (EVLW) of a patient after lung resection, adapted to provide a transpulmonary thermodilution curve, capable to derive the difference between the patient's intrathoracic thermal volume (ITTV) and approximated intrathoracic blood volume (ITBV) from the transpulmonary thermodilution curve, and capable to determine the extravascular lung water volume (EVLW) by correcting by the degree of lung resection (c), wherein making use of the equation

$$EVLW = f(ITTV,\ ITBV_{approx},\ c),$$

or

$$EVLW = f(ITTV,\ GEDV,\ c).$$

2. Apparatus according to claim 1, wherein the apparatus is adapted to estimate the degree of lung resection (c) by making use of an empirical average value of the reduction of pulmonary perfusion of the resected lung compared to the pre-operative lung of the patient.

3. Apparatus according to claim 1, wherein in case of a resected right lung the apparatus is adapted to estimate the degree of lung resection (c) as being 55%, or in case of a resected left lung the apparatus is adapted to estimate the degree of lung resection (c) as being 45%, and/or in case of a resected lobes, estimating the degree of lung resection c as being the sum of normal perfusion rates of the resected lobes.

4. Apparatus according to claim 1, wherein the apparatus is adapted to estimate the degree of lung resection (c) by comparing the pulmonary perfusion before and after the lung resection, wherein the pulmonary perfusion values are provided by making use of pre-operative perfusion lung scans.

5. Apparatus according to any of claims 1 to 4, wherein the apparatus is adapted to make use of the equation

$$EVLW = ITTV - ITBV|_{corrected},$$

wherein $ITBV|_{corrected}$ is the value of the approximated intrathoracic blood volume ($ITBV_{approx}$) corrected by the degree of lung resection (c).

6. Apparatus according to any of claims 2 to 5, wherein the apparatus is adapted make use of equating the degree of lung resection (c) with the degree of reduction of pulmonary blood volume (PBV) caused by the lung resection.

7. Apparatus according to claim 6, wherein the apparatus is adapted to derive the global end-diastolic volume (GEDV) of the patient from the transpulmonary thermodilution curve and is adapted to make use of the equation

$$ITBV|_{corrected} = GEDV + PBV|_{corrected},$$

wherein $PBV|_{corrected}$ is the value of the pulmonary blood volume (PBV) corrected by the degree of lung resection (c).

8. Apparatus according to claim 7, wherein the apparatus is adapted to make use of the equation

$$PBV|_{corrected} = ((a - 1)\ GEDV + b)\ (100\% - c[\%]),$$

wherein a and b are parameters.

9. Apparatus according to claim 8, wherein the parameters a and b are set to be
a = 1.25 and b = 0.0.

10. Computer program for assessing extravascular lung water volume (EVLW) of a patient after lung resection, having instructions adapted to carry out the steps:

   - generating a transpulmonary thermodilution curve on basis of provided measurement data collected at the patient,
   - deriving the patient's intrathoracic thermal volume (ITTV) and approximated intrathoracic blood volume ($ITBV_{approx}$) from the transpulmonary thermodilution curve, and
   - determining the extravascular lung water volume (EVLW) by correcting by the degree of lung resection (c), wherein making use of the equation

$$EVLW = f(ITTV,\ ITBV_{approx},\ c),$$

   or

$$EVLW = f(ITTV,\ GEDV,\ c),$$

   when run on a computer.

11. Computer program according to claim 10, having instructions adapted to carry out the step:

   - estimating the degree of lung resection (c) by making use of an empirical average value of the reduction of pulmonary perfusion of the resected lung compared to the pre-operative lung of the patient.

**12.** Computer program according to claim 10, having instructions adapted to carry out the steps:

in case of a resected right lung

- estimating the degree of lung resection (c) as being 55%,

or in case of a resected left lung

- estimating the degree of lung resection (c) as being 45%,

and/or in case of resected lobes

- estimating the degree of lung resection (c) as being the sum of normal perfusion rates of the resected lobes.

**13.** Computer program according to claim 10, having instructions adapted to carry out the step:

- estimating the degree of lung resection (c) by comparing the pulmonary perfusion before and after the lung resection, wherein the pulmonary perfusion values are provided by making use of pre-operative perfusion lung scans.

**14.** Computer program according to any of claims 11 to 13, having instructions adapted to carry out the step:

- making use of the equation

$$EVLW = ITTV - ITBV\big|_{corrected},$$

wherein $ITBV\big|_{corrected}$ is the value of the approximated intrathoracic blood volume ($ITBV_{approx}$) corrected by the degree of lung resection (c).

**15.** Computer program according to any of claims 11 to 14, having instructions adapted to carry out the step:

- making use of equating the degree of lung resection (c) with the degree of reduction of pulmonary blood volume (PBV) caused by the lung resection.

**16.** Computer program according to claim 15, having instructions adapted to carry out the steps:

- deriving the global end-diastolic volume (GEDV) of the patient from the transpulmonary thermodilution curve and
- making use of the equation

$$ITBV\big|_{corrected} = GEDV + PBV\big|_{corrected},$$

wherein $PBV\big|_{corrected}$ is the value of the pulmonary blood volume (PBV) corrected by the degree of lung resection (c).

**17.** Computer program according to claim 16, having instructions adapted to carry out the step:

- making use of the equation

$$PBV\big|_{corrected} = ((a - 1)\ GEDV + b)\ (100\% - c[\%]),$$

wherein a and b are parameters.

**18.** Computer program according to claim 17, having instructions adapted to carry out the step:

- setting the parameters a and b to be
a = 1.25 and b = 0.0.

FIG. 1

| | European Patent Office | EUROPEAN SEARCH REPORT | Application Number EP 07 00 8877 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SCHRÖDER ET AL: "Radical Lymphadenectomy Does Not Effect Intrathoracic Fluid Volume Changes After Lung Surgery" THE INTERNET JOURNAL OF THORACIC AND CARDIOVASCULAR SURGERY, [Online] vol. 7, no. 2, 2005, XP002452454 Retrieved from the Internet: URL:http://www.ispub.com/ostia/index.php?xmlPrinter=true&xmlFilePath=journals/ijtcvs/vol7n2/radical.xml> [retrieved on 2007-09-25] * the whole document * | 1-4, 10-13 | INV. A61B5/028 |
| Y | | 2-9, 11-18 | |
| X | ROCH ANTOINE ET AL: "Accuracy and Limits of Transpulmonary Dilution Methods in Estimating Extravascular Lung Water After Pneumonectomy" August 2005 (2005-08), CHEST AUG 2005, VOL. 128, NR. 2, PAGE(S) 927 - 933 , XP002452457 ISSN: 0012-3692 * page 928, column 1, line 21 - line 23 * * page 931, column 1, line 13 - line 26 * * page 931, column 2, line 7 - line 11 * * page 933, column 1, line 45 - line 48 * | 1,10 | |
| Y | | 2-9, 11-18 | TECHNICAL FIELDS SEARCHED (IPC) A61B |
| X | LEE ET AL: "Effect on Pneumonectomy on Extravascular Lung Water in Dogs" JOURNAL OF SURGICAL RESEARCH, vol. 38, no. 6, 1985, pages 568-573, XP008083691 * page 570, column 1, line 6 - line 10 * -/-- | 1,10 | |

The present search report has been drawn up for all claims

| Place of search The Hague | Date of completion of the search 25 September 2007 | Examiner Bengtsson, Johan |
|---|---|---|

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**European Patent Office**

**EUROPEAN SEARCH REPORT**

Application Number

EP 07 00 8877

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SAKKA ET AL: "Assessment of cardiac preload and extravascular lung water by single transpulmonary thermodilution" INTENSIVE CARE MEDICINE, vol. 26, no. 2, 2000, pages 181-187, XP002452455 * page 185, column 2, line 12 - line 31 * ----- | 5-9, 14-18 | |

TECHNICAL FIELDS
SEARCHED (IPC)

The present search report has been drawn up for all claims

| Place of search | Date of completion of the search | Examiner |
|---|---|---|
| The Hague | 25 September 2007 | Bengtsson, Johan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5526817 A